# EUROPÄISCHE PATENTANMELDUNG

(11) **EP 0 676 466 A2**
(43) Veröffentlichungstag der Anmeldung: **11.10.1995**
(21) Anmeldenummer: 95102642.6
(22) Anmeldetag: 24.02.1995
(51) Int. Cl.: C12M 1/20

(54) **Feststoff-Bioreaktor**

(30) Priorität: 01.03.1994 DE 4406632
(71) Anmelder: Vollbrecht, Dietmar, Dr., D-37133 Friedland, OT Gross Schneen (DE)
(72) Erfinder: Vollbrecht, Dietmar, Dr., D-37133 Friedland, OT Gross Schneen (DE)

(57) **Zusammenfassung**

Feststoff-Bioreaktor zur Kultur von Mikroorganismen auf festen, partikulären Substraten dadurch gekennzeichnet, daß die Temperatur zwischen -27 und +100^{o}C, die relative Feuchte vermittels von mit Ultraschall erzeugtem, kaltem Wasserdampf von 40-99% regelbar ist, daß eine gerichtete, in der Zusammensetzung dosierbare (Sauerstoffgehalt 0 bis 100% im Gemisch mit Stickstoff, Kohlendioxid und gegebenenfalls weiteren Gasen einstellbar) und im Volumenstrom regelbare Zwangsströmung der Gasatmosphäre erzeugt wird, daß Einrichtungen zur Heißdampfsterilisation des Bioreaktors sowie solche zur Ableitung und Sterilisation von Kondensat und Abluft vorhanden sind.

## Beschreibung

Die Erfindung betrifft einen Feststoff-Bioreaktor zur Kultur von Mikroorganismen auf festen, gequollenen, partikulären Substraten unter kontrollierten Bedingungen (Temperatur, Gasatmosphäre, Feuchtigkeit).

Verschiedene Konzepte zur Auslegung und Gestaltung von Feststoff-Bioreaktoren sind bekannt. Zu den am häufigsten in der Fachliteratur diskutierten Typen zählt der sogenannte "Rotary Drum Reaktor". Dieser besteht aus einem auf der Seite liegenden, hohlen Zylinder, in dessen Innenraum Mikroorganismen auf festen Substraten kultiviert werden sollen. Da das Substrat sich in diesem Bioreaktortyp in einer relativ dicken Schicht befindet, muß es zur Aufrechterhaltung einer ausreichenden Ventilation (Abführen der Oxidationswärme, Entfernung von überschüssigem Kohlendioxid, ausreichende Versorgung mit Sauerstoff sowie Feuchtigkeit) während der Fermentation ständig oder in regelmäßigen Intervallen umgewälzt werden. Ein wesentlicher, hiermit verbundener Nachteil ist jedoch, daS das an der Oberfläche des partikulären Substrates befindliche Pilzmycel durch die bei der Umwälzung auftretenden Scherkräfte geschädigt wird. Hierdurch wird die Stoffwechselaktivität der betreffenden Kultur stark vermindert, oder sie kommt sogar zum Stillstand, bevor sie sich nach mehreren Stunden ohne Umwälzung allmählich wieder erholen kann. Die Rotary Drum Reaktoren haben sich wegen dieses Nachteils nicht auf dem Markt behaupten können.

Es wurde daher versucht, ruhende, statische Kulturen grosser Schichtdicke (bis 1,50 m) zu kultivieren. Hiermit ist jedoch der Nachteil verbunden, daS wegen der hohen Schichtdicke erhebliche Diffusionswiderstände auftreten. Diese lassen sich nur durch das Einblasen von Luft oder anderen, geeigneten Gasgemischen unter Druck überwinden. Außerdem vergrößert sich bei fortschreitendem Mycelwachstum der Diffusionswiderstand infolge des Zuwachsens der interpartikulären Räume derart, daß vor Erreichen des maximal möglichen Wachstums der Prozeß wegen Überhitzung, Mangel an Kohlendioxidventilation sowie Mangel an Sauerstoffversorgung zusammenbricht und daher vorzeitig abgebrochen werden muß.

Bei einer weiteren Variante wird die Feststoff-Fermentation in einer relativ dünnen Schicht, auf flachen Tabletts, durchgeführt. Hierdurch werden die für die anderen beiden Typen beschriebenen Nachteile vermieden, jedoch tritt ein anderer Nachteil auf; denn wegen des auf viele flache Tabletts verteilten, in Abständen installierten Substrates sinkt die auf das Gesamtvolumen des Bioreaktors bezogene Nutzlast erheblich. Ein weiterer Nachteil der bisher für die Kultur in flachen Tabletts verwendeten Bioreaktoren ist, daß die Oxidationswärme durch eine entsprechende Intensität der Durchströmung des Reaktorraumes mit angefeuchteter, steriler Luft kompensiert wird. Mit zunehmendem Wachstum führt dies bei, wie in der Praxis üblich, nur geringfügig geringeren Temperatur der Zuluft im Reaktionsraum zu notwendigen Luftwechselraten von bis mehr als 100 Luftwechseln/min. Hierdurch werden jedoch Überströmungsgeschwindigkeiten erzeugt, bei denen das an der Oberfläche befindliche Mycel mit signifikant verringerter Stoffwechselaktivität nach Art von Wetterfichten wächst. Dies ist jedoch im Interesse einer optimalen Prozeßführung unerwünscht. Ein weiterer Nachteil der für die Kultur in flachen Tabletts bisher verwendeten Bioreaktoren ist, daß, insbesondere bei größeren Anlagen unter Vollast und intensivem Wachstum, erhebliche Mengen an geruchsintensiver und keimbelasteter Abluft entsorgt werden müssen. Ein weiterer Nachteil der für die Kultur in flachen Tabletts verwendeten Bioreaktoren ist, daß der Dampf für die Befeuchtung der eingeblasenen Luft bzw. des Gasgemisches in Erhitzern erzeugt wird. Hierdurch sind erhebliche Anstrengungen zum Herunterkühlen des heißen Dampfes bedingt. Beim Herunterkühlen geht jedoch ein großer Teil des Wasserdampfes durch Kondensation wieder verloren. Dies bedingt einen relativ geringen Wirkungsgrad und eine hieraus resultierende überdimensionale Anlage zur Luftbefeuchtung. Wegen dieser Nachteile wird daher, wie bereits erwähnt, in der Regel die einzublasende, befeuchtete Luft auf eine Temperatur abgekühlt, die nur unwesentlich unterhalb der gewünschten Reaktorinnentemperatur liegt. Dies bedingt einen relativ geringen Wirkungsgrad bei der notwendigen Ableitung von Oxidationswärme.

Es ist deshalb Aufgabe der Erfindung, einen Bioreaktortyp zur Kultur von Mikroorganismen auf festen, partikulären Substraten aufzuzeigen, der die genannten Nachteile nicht aufweist.

Erfindungsgemäß wird diese Aufgabe gelöst durch eine Vorrichtung, die die Merkmale des Patentanspruchs 1 enthält. Eine bevorzugte Ausgestaltung stellt eine Vorrichtung gemäß Anspruch 2 dar.

Der Feststoff-Bioreaktor nach der vorliegenden Erfindung wird nun an verschiedenen Beispielen erläutert.

### Beispiel 1:

In dem erfindungsgemäßen Bioreaktor waren Gasaustausch und Wachstum einer Kultur von Aspergillus niger bei einer Neigung der Tabletts von 15^{o} gegenüber anderen auf dem Markt befindlichen Systemen wesentlich beschleunigt: das Wachstum war nach ca. 24 h optimal, verglichen mit 48 bis 72 h bei anderen Systemen. Dies ist eine Verbesserung der Durchsatzleistung um mehr als 100%. Infolge der verbesserten Ventilation konnten im Vergleich zu anderen Systemen die lichten Abstände zwischen den übereinander liegenden Tabletts auf nur 1 bis 4 cm gegenüber sonst 5 bis 15 cm und mehr begrenzt werden, und die Nutzlast-Kapazität gegenüber anderen Systemen wurde um mehr als 35% gesteigert.

### Beispiel 2:

In dem erfindungsgemäßen Bioreaktor betrug die Zeitspanne bis zum Erreichen des maximalen Wachstums bei Rhizopus oligosporus 15 - 20 h gegenüber 48 - 72 h bei anderen Systemen mit weniger effizienter Ventilation, die zur Verbesserung derselben auch noch in periodischen Abständen durchmischt werden müssen. Dies ist eine Verbesserung der Durchsatzleistung um über 100%.

### Beispiel 3:

Infolge der regelbaren Umwälzung der Gasatmosphäre innerhalb des erfindungsgemäßen Feststoff-Bioreaktors zur Vermeidung turbulenter Strömungen über dem bewachsenen Substrat ließen sich, verglichen mit anderen Systemen, die Geschwindigkeit des Wachstums, die Produktbildungsrate sekundärer Metaboliten sowie die Ausbeute an Biomasse bzw. Produkt in Kulturen mit Penicillium chrysogenum um ca. 25% steigern.

### Beispiel 4:

Die Gestaltung der Inneneinrichtung des erfindungsgemäßen Feststoff-Bioreaktors mit plattenförmigen, flachen Wärmetauschern sowie Leitblechen zur Erzeugung einer gerichteten Zwangsströmung führte in Kulturen mit Rhizopus oligosporus zu einer Verringerung der für eine ausreichende Ventilation erforderlichen Umwälzleistung sowie der erforderlichen Frischluftversorgung um jeweils ca. 50%, verglichen mit anderen Systemen.

### Beispiel 5:

Die dosierte Zuspeisung von befeuchteter Frischluft in dem erfindungsgemäßen Feststoff-Bioreaktor zur Kompensation des durch eine Kultur von Aspergillus oryzae verbrauchten Sauer- stoffs sowie zur Ventilation des produzierten Kohlendioxids ergab, verglichen mit der sonst üblichen Frischluftversorgung, eine Verringerung des geruchsintensiven und keimbelasteten Abluftvolumens um ca. 80%. Hierdurch wird, verglichen mit anderen Systemen, eine signifikante Kostensenkung bei der Entsorgung der Abluft sowie eine entsprechende Senkung der Emissionen erreicht.

### Beispiel 6:

Die dosierte Zuspeisung von befeuchtetem Sauerstoff in dem erfindungsgemäßen Feststoff-Bioreaktor zur Optimierung der Sauerstoffversorgung für die Produktion sekundärer Metaboliten in einer Kultur von Penicillium chrysogenum ergab bei einer Sättigung des Sauerstoffbedarfs der Kultur von ca. 80% eine Ausbeutesteigerung von 45% verglichen mit einer reinen Variation des Frischluftdurchsatzes.

### Beispiel 7:

Das Wachstum sowie der Substratausbeutekoeffizient von verschiedenen Schimmelpilzen wurde in einer definierten Gasatmosphäre bestehend aus Stickstoff (N₂), Sauerstoff (O₂) und Kohlendioxid (CO₂) durch Partialdrücke von CO₂ zwischen 3-5% um ca. 25% gegenüber entsprechenden, optimal belüfteten Kulturen gefördert.

### Beispiel 8:

Die Einspeisung von durch einen Ultraschallgenerator erzeugtem Kaltdampf, ergab, verglichen mit der sonst üblichen Einspeisung von heruntergekühltem Heißdampf, in dem erfindungsgemäßen Feststoff-Bioreaktor eine Energieeinsparung von 70%.

### Beispiel 9:

Infolge des im voranstehenden Beispiel beschriebenen, verringerten Wärmeeintrages ließ sich die Nutzlast des erfin- dungsgemäßen Feststoff-Bioreaktors mit einer Kultur von Aspergillus oryzae um den Faktor 2,5, d.h. um 250%, im Vergleich zu anderen Systemen steigern.

### Beispiel 10:

Die Ausbildung ausgedehnter, flacher, plattenförmiger Wärmeaustauscherflächen anstelle einfacher Leitbleche zur Erzeugung eines gerichteten Luftstromes bei dem erfindungsgemäßen Bioreaktors ermöglicht beim Überschreiten der Maximaltemperatur innerhalb enger Temperaturgrenzen von ±1^{o}C eine leistungsfähige Kühlung, bei der die Temperatur der Wärmeaustauscherflächen nur ca. 2^{o}C unterhalb der erwünschten Fermentationstemperatur liegt. Hierdurch wird innerhalb des erfindungsgemäßen Feststoff-Bioreaktors ein unerwünschtes Austrocknen der Gasatmosphäre weitgehend vermieden. Wie Vergleiche von Kulturen mit Aspergillus, Penicillium, Rhizopus, Mucor ssp. mit solchen in herkömmlichen Feststoff-Bioreaktoren ergaben, wurden in dem erfindungsgemäßen Feststoff-Bioreaktor bei maximaler Auslastung Wachstum bzw. Produktbildung infolge der verbesserten Konstanthaltung von Temperatur und Feuchtigkeit innerhalb eines sehr engen, optimalen Bereiches (±1^{o}C bzw. ±1% r.H.) um 20 bis 30% gesteigert.

### Beispiel 11:

Infolge der im Vergleich zu thermischen Bedampfungssystemen in anderen Feststoff-Bioreaktoren effizienteren Nutzung der Bedampfungskapazität lassen sich bei dem erfindungsgemäßen Feststoff-Bioreaktor die installierte Bedampfungskapazität um ca. 60% sowie die betreffenden Installationskosten um etwa denselben Prozentsatz reduzieren.

### Beispiel 12:

Infolge der Einhaltung der relativen Feuchtigkeit, einer in diesem speziellen Fall sehr kritischen Größe, in dem erfindungsgemäßen Feststoff-Bioreaktor innerhalb relativ enger Grenzen (95 ±1%) wurde die Fermentationsdauer von Monascus purpureus zur Erzeugung des purpurroten Pigmentes auf sechs Tage verkürzt, verglichen mit 15 bis 21 Tagen in anderen Systemen.

### Beispiel 13:

Untersuchungen zur Simulation der mikrobiell bedingten und volkswirtschaftlich nicht unbedeutenden Selbsterhitzung des Heus wurden in dem erfindungsgemäßen Feststoff-Bioreaktor bis zu einer Temperatur von 95^{o}C durchgeführt. Bei diesen Versuchen wurde die Temperatur des Bioreaktors derjenigen im lose gepackten Heu nachgeführt, um die Wärmeverluste der Heupackung an die Umgebung zu minimieren. Feuchtigkeit und Luftzufuhr wurden kontrolliert. Auf diese Weise wurden das Maximum des Wachstums thermophiler Mikroorganismen (Bacillus stearothermophilus sowie Thermoactinomyces vulgaris) und der Beginn der sogenannten pyrogenen Phase (ab ca. 95^{o}C) bereits nach 2-3 Tagen erreicht. Hieraus ergibt sich, daß der erfindungsgemäße Feststoff-Bioreaktor eine den Verhältnissen in Scheunen (2-3 Tage) stärker angenäherte Simulation der mikrobiellen Vorgänge bei der Selbsterhitzung des Heus erlaubt als die bisher speziell hierfür ausgelegten Vorrichtungen (7 bis 13 Tage, Fehlen definierter Feuchtigkeit und definierter Versorgung mit Luft bzw. Sauerstoff). Derartige Untersuchungen sind mit anderen Feststoff-Bioreaktoren aufgrund von deren eingeschränkten Arbeitsbereichen nicht möglich.

### Beispiel 14:

Der Ligninabbau von vermahlenem, pelletierten und mit den üblichen mineralischen Nährstoffen beaufschlagtem Holzmehl durch Phanaerochaete chrysosporium betrug in dem erfindungsgemäßen Bioreaktor nach 6-7 Tagen 70 Prozent, verglichen mit Zeiträumen zwischen 30 und 60 Tagen in anderen Feststoff-Fermentationen bis zum Erreichen eines vergleichbaren Abbaugrades.

## Patentansprüche

1. Feststoff-Bioreaktor zur Kultur von Mikroorganismen auf festen, partikulären Substraten
**dadurch gekennzeichnet**, daß
- im Innenraum in einem Winkel von 10-15^{o} geneigte Tabletts angeordnet sind,
- die Versorgung der Kultur mit einer definierten Gasatmosphäre durch eine strömungs- bzw. druckgeregelte Begasungseinrichtung erfolgt,
- die Befeuchtung des Gasvolumenstromes auf eine relative Feuchte zwischen 40 und 99% durch eine Vorrichtung zur Erzeugung von kaltem Wasserdampf vermittels hochfrequenter Schwingungen erfolgt,
- im Inneren eine Vorrichtung zur Luftumwälzung angeordnet ist, und die Luft bzw. das Gasgemisch in einer durch Leitbleche zwangsweise geführten Strömung und mit geringer Strömungsgeschwindigkeit von der tiefer gelegenen Seite des Tabletts an der Ober- und Unterseite des Tabletts entlang zu dessen höher gelegener Seite streicht,
- die Temperatur auf Werte zwischen -27^{o}C und +100^{o}C einstellbar ist,
- zur Abführung der Oxidationswärme an den Leitblechen Wärmeaustauscher vorgesehen sind, deren Temperatur 2-3^{o}C unterhalb der gewünschten Fermentationstemperatur liegt,
- zur Heißdampf-Sterilisation des Feststoff-Bioreaktors eine eigene, drucklose Heißdampfversorgung oder der Anschluß an eine externe, drucklose Heißdampfversorgung vorgesehen ist,
- zur Ableitung von Kondensat am tiefsten Punkt des Reaktor-Innenraumes ein Ablauf mit einer hitzesterilisierbaren Falle installiert ist,
- zur Ableitung der Abluft oberhalb des höchsten Tabletts eine hitzesterilisierbare Vorrichtung zum Ableiten, Sterilisieren und, gegebenenfalls, Desodorieren der geruch- und keimbelasteten Abluft installiert ist,

2. Feststoff-Bioreaktor nach Anspruch 1), dadurch gekennzeichnet, daß die Temperatur bevorzugt auf Werte zwischen +20^{o}C und +45^{o}C einstellbar ist.
